Europäisches Patentamt

European Patent Office

Office européen des brevets

(19)

(11) Publication number: **0 444 303 A2**

# EUROPEAN PATENT APPLICATION

(21) Application number: 90125247.8

(22) Date of filing: **21.12.90**

(51) Int. Cl.⁵: **G01N 33/569**, G01N 33/571, G01N 33/543

(30) Priority: **01.03.90 US 486998**

(43) Date of publication of application:
**04.09.91 Bulletin 91/36**

(84) Designated Contracting States:
**AT BE CH DE DK ES FR GB GR IT LI LU NL SE**

(71) Applicant: **Becton Dickinson and Company
One Becton Drive
Franklin Lakes New Jersey 07417-1880(US)**

(72) Inventor: **McLaurin, Daniel A., III
912 Vickie Drive
Cary, North Carolina(US)**
Inventor: **Pearson, Robert E.
5507 Loyal Avenue
Durham, North Carolina(US)** ·
Inventor: **Hopkins, Anne C.
105 Seth Court
Cary, North Carolina(US)**
Inventor: **Scott, Patricia B.
119 Trappers Run Drive
Cary, North Carolina(US)**
Inventor: **Edelstein, Susan B.
308 Heidinger Drive
Cary, North Carolina(US)**
Inventor: **Myatich, Ronald G.
500 18A Woodcroft Parkway
Durham, North Carolina(US)**
Inventor: **Mapes, James P.
8005 Kingsland Drive
Raleigh, North Carolina(US)**

(74) Representative: **von Kreisler, Alek,
Dipl.-Chem. et al
Patentanwälte Von Kreisler-Selting-Werner,
Deichmannhaus am Hauptbahnhof
W-5000 Köln 1(DE)**

(54) Detection of chlamydia by flow-through assay.

(57) A method for flow-through assay of Chlamydia includes digesting a sample suspected of containing Chlamydia with Proteinase K and passing the digested sample through a microporous membrane having an anti Chlamydia antibody adjacent an absorbent pad which promotes flow through the membrane by capillary action. Antigen bound to antibody on the membrane may be contacted with a specific anti Chlamydia monoclonal antibody conjugated to an enzyme and washed with a solution containing a chaotropic agent . Passage of a substrate for the enzyme through the membrane causes a detectable colored spot to appear on the membrane if the sample is positive for Chlamydia. The invention includes a reagent test kit for performing the assay.

## BACKGROUND OF THE INVENTION

### 1. Field of the Invention.

This invention relates to an assay for an analyte, and more specifically relates to an improved membrane immunoassay for Chlamydia.

### 2. Background of the Invention.

The genus Chlamydiaceae includes two species, Chlamydia trachomatis and Chlamydia psittaci. Chlamydia trachomatis in its some fifteen various strains is the etiologic agent for a number of human ocular and genital diseases, including trachoma, inclusion conjunctivitis, lymphogranuloma venereum "non-specific" or nongonococcal urethritis and proctitis. C. trachomatis infection is pervasive throughout the general population. It has been estimated, for instance, that C. trachomatis is accountable for several million cases per year of nongonococcal urethritis. Since C. trachomatis-mediated disease is widespread, a reliable, simple and inexpensive test for the organism's presence is highly desirable and of great importance so that proper treatment can be undertaken.

Several immunoassay procedures for Chlamydia have been disclosed. PCT-published application number WO 86/02733 discloses an assay for various antigens including Chlamydia which cause ocular infections. The assay includes immobilizing the antigen on a solid support by binding to a monoclonal antibody absorbed on the support, or the antigen is absorbed directly onto the solid support.

European Patent Application Number 0183383 discloses an assay for Chlamydia antigen which includes an isolation procedure in which the antigen is heated to a temperature of about $100^\circ$ C to reduce nonspecific binding.

Rose, in U.S. Patent No. 4,663,291, discloses treating a specimen suspected of containing Chlamydia organisms with a surfactant and a metal ion to release Chlamydia antigen, followed by assay of the antigen by known methods. In accordance with the method, the known inhibition of binding of the antigen to anti Chlamydia antibody by the surfactant does not occur.

Armstrong et al. in U.S. Patent No. 4,497,899, discloses an immunoassay for Chlamydia antigen in which a sample suspected of containing Chlamydia organisms is lysed to release the antigen which is absorbed directly onto a solid support.

A Chlamydia assay kit is marketed under the name ChlamydiazymeTM.

Immunoassays conducted on a microporous membrane are often referred to as flow-through assays since the assay steps are performed sequentially with the fluid phase of each step passing through the membrane before the next step is initiated. Flow-through assay devices in which flow is enhanced by capillary action induced by an absorbent pad in contact with the membrane are disclosed by U.S. Patent Nos. 3,888,629 to Bagshaw and 4,632,901 to Valkirs et al.

A problem which may arise in flow-through assay devices is absorption of various components of a test solution other than antigen onto the membrane. This problem may lead to other problems, such as partial plugging of the membrane, impeded fluid flow and nonspecific absorption of assay tracer to the absorbed components.

In spite of the above disclosures, there yet remains a definite need for further improvement in assays for Chlamydia. This invention is directed toward fulfillment of this need by providing a membrane immunoassay for Chlamydia.

## SUMMARY OF THE INVENTION

A method for flow-through assay of Chlamydia includes digesting a clinical sample with Proteinase K and passing the digested sample through a microporous membrane coated with anti Chlamydia antibodies to cause Chlamydia antigen in the sample to adhere to the membrane. The membrane having absorbed antigen is treated with a chaotropic agent and a tracer including an antibody conjugated to an enzyme. Enzyme captured on the membrane is reacted with a substrate to cause appearance of a colored spot on the membrane. A preferred assay includes filtering the digested sample prior to passing it through the coated membrane and may also include washing the membrane with an oxidizing agent to decolorize any blood present in sample. In the most preferred assay, the tracer is an anti Chlamydia monoclonal antibody conjugated to alkaline phosphatase (AP) and the substrate is indoxyl phosphate.

The invention includes a kit of materials for performing the assay method of the invention.

In studying the application of flow-through assay to Chlamydia, it has been found that the procedure is complicated by several problems which do not arise in Chlamydia assays performed by other methods or in flow-through assay for other analytes. Thus, it has been found that clinical samples containing Chlamydia antigen, even when there are no undissolved components, do not consistently flow through the membrane. In most cases, this causes at least some impedance of flow, and in extreme cases, full blockage of flow results. Even in those cases where flow is not completely blocked, interference with binding of antigen to the trapping antibody may occur to a sufficient extent to cause some positive samples to appear negative because insufficient antigen was absorbed to bind to tracer.

In accordance with the invention, it has been found that clinical samples treated with Proteinase K flow easily through the membrane with complete binding of antigen to trapping antibody. Proteinase K, however, while assuring good flow, may not alone provide an assay of high sensitivity and specificity because it does not prevent other components which may nonspecifically bind to tracer from being absorbed on the membrane. Thus, Proteinase K alone may not prevent an unacceptably high level of false positives. When Proteinase K digestion of the clinical sample prior to binding is combined with a post binding treatment with a chaotrope, flow proceeds normally, a very low level of false positives occurs, and true positives are easily recognized by a deeply colored spot on the membrane.

## DETAILED DESCRIPTION

While this invention is satisfied by embodiments in many different forms, there will herein be described in detail preferred embodiments of the invention, with the understanding that the present disclosure is to be considered as exemplary of the principles of the invention and is not intended to limit the invention to the embodiments described. The scope of the invention will be measured by the appended claims and their equivalents.

Clinical samples generally contain many components other than the analyte to be measured. In some cases, these components, because of their size or chemical composition, may change the character of the sample, even though all are soluble, such that flow of the sample through a microporous membrane may be impeded, or, in extreme cases, the membrane may become plugged. In other cases, components of a clinical sample other than the analyte may adhere to the membrane and then bind nonspecifically to the tracer giving rise to false positives.

It has now been discovered that clinical samples suspected of containing an analyte which must be detected before proper treatment of a patient can begin may be prepared for flow-through assay by prior treatment with the enzyme Proteinase K. It is believed, although not substantiated, that Proteinase K digests various components of the sample, thereby altering the sample and facilitating flow through the membrane.

While the invention contemplates treating any clinical sample intended for flow-through assay with Proteinase K, the method will be described in detail for assay of Chlamydia. A sample suspected of harboring a Chlamydia infection may be taken from a patient's body by any conventional means. Preferably a swab sample is taken, most preferably a genital sample, and suspended in a transport liquid, such as water or a buffer, by agitating the swab to release the Chlamydia organisms and free antigen into the liquid. While any transport liquid which aids in release of antigen from the swab may be used, such as dilute acetic acid, a preferred transport liquid is 0.2 M sucrose in phosphate buffer.

The preferred antigen of the invention is a Chlamydia cell surface lipopolysaccharide (LPS), and accordingly, the assay is preferably performed with Chlamydia organisms. However, if it is desired to assay for an antigen which is not exposed on the cell surface, the organisms may be pretreated to achieve greater exposure of the antigen. Any reagent or technique which alters the Chlamydia cells in a manner which renders the antigen more available for binding to the capture antibody and tracer may be used. For example, a surfactant may be used to disrupt the Chlamydia organisms, or a conventional technique such as osmotic lysis or sonication may be used.

It has been found that the Chlamydia assay of the invention is more sensitive and more specific if the antigen sample is digested with Proteinase K prior to adding the sample to the membrane. Digestion with Proteinase K is preferably performed after diluting the sample in the transport liquid with a buffer, such as Tris or preferably 2-(N-morpholino)-ethanesulfonic acid, (MES) pH 6. A suitable concentration of the MES buffer is about 50-200, preferably about 100 mM.

A solution of Proteinase K in Acetate Buffer may be added to the MES diluted sample to give a concentration of about 0.1 to 4, preferably about 0.5 to 2, most preferably about 1 mg/ml. The sample containing Proteinase K may then be maintained at about 25 to $50^\circ$C, preferably about $25^\circ$C for about 5 to 30, preferably about 10 minutes.

After digestion with Proteinase K, the sample is preferably filtered. Any filtering medium, such as a cotton plug, may be used; however, it is preferable to pass the digested sample through a suitable filter

3

such as Whatman #1, Whatman #41, Gelman V-M1, or most preferably through a DuraporeTM polyvinylidene fluoride filter, available from Millipore Corp., Bedford, Massachusetts.

The antigen in the filtrate may be absorbed onto a membrane. Preferably, the membrane may be precoated with anti Chlamydia capture antibody, preferably a polyclonal antibody, and an inert protein. In the present disclosure, the term inert protein means a protein which is immunologically unreactive toward any other component of the assay and which does not substantially bind nonspecifically to other proteins in the assay medium, with the understanding that the inert protein may well be immunologically reactive toward other materials which are not part of the assay system. The inert protein serves to fill any unreacted binding sites on the membrane. Suitable inert proteins are, for example, albumin or casein.

Preferably, the membrane is positioned over an absorbent pad or filter stack which generates flow of liquid through the membrane by capillary action. Any membrane which absorbs the Chlamydia antigen in the filtered sample may be used. Suitable membranes are polycarbonate, nitrocellulose and nylon and are well known in the art. A particularly preferred membrane is a nylon based BiodyneTMA membrane (Pall Corp., Glen Cove, New York). The membrane and absorbent pad may be mounted in a housing having an aperture for access to the membrane. Any suitable device for flow-through assay by capillary action may be used. Several such devices are commercially available.

Binding of Chlamydia antigen onto the membrane takes place readily and is complete by the time the liquid has passed through the membrane.

Some Chlamydia samples contain blood which may color the membrane and cause a Chlamydia negative sample to appear positive. This possible source of false positives may be eliminated by a particular antigen wash. Accordingly, after the filtered sample has been applied to the membrane and allowed to drain through, a wash solution containing an oxidizing agent may be passed through the membrane. A preferred oxidizing agent is an aqueous solution of hydrogen peroxide of about 10 to 20, preferably about 15% by volume concentration. The most preferred oxidizing solution additionally contains about 2.5 to 8, preferably about 5% by volume each of butanol and acetic acid. Applicants have found that these two reagents contribute to enhancement of the color signal from positive samples.

The membrane containing absorbed antigen may also be treated with a chaotropic agent. Such agents are known to disrupt noncovalent bonds such as hydrogen bonds and electrostatic bonds and thereby weaken molecular interactions. Thus, a feature of the present invention is passage of a concentrated solution of a suitable chaotropic agent, such as guanidine, hydrochloride, sodium chloride, potassium iodide, potassium thiocyanate or preferably urea, through the membrane having absorbed antigen. This treatment is believed to disrupt protein-protein interactions which survived the Proteinase K treatment so that complex molecules other than Chlamydia antigen are removed from the membrane. Substantial reduction in nonspecific binding is thereby accomplished and the appearance of false positives is greatly reduced. In a preferred treatment, a solution of urea of about 2 to 8, preferably about 8M at a pH of about 10 to 14, preferably about 13, may be passed through the membrane.

Subsequent to washing the membrane with the oxidizing and chaotropic solutions, the absorbed Chlamydia antigen is contacted with a tracer. The tracer may be an anti Chlamydia antibody conjugated to an enzyme. Preferably, the tracer is in a suitable buffer compatible with the tracer.

The most preferred tracer includes an anti-Chlamydia monoclonal antibody which has been raised against Chlamydia cell surface lipopolysaccharide by conventional fusion and selection techniques. Procedures for raising monoclonal antibodies and conjugating an enzyme to an antibody are well-known and no further details are needed for a complete understanding of this aspect of the invention by one skilled in the art.

A suitable enzyme is a peroxidase, such as horseradish peroxidase or, preferably, a hydrolase such as, for example, a peptidase, esterase, glycosidase, such as galactosidase, or most preferably, a phosphatase such as AP.

Binding between antigen and tracer on the membrane is generally complete in the time, about 15-30 seconds, it takes for the tracer solution to drain through the membrane. After binding, the membrane may be washed with a buffer, optionally containing urea. A preferred wash buffer is a trizma (tris(hydroxy-methyl)amino methane) buffer containing about 4 M urea.

Suitable substrates for reaction with AP captured on the membrane are phosphates such as naphthyl phosphates which form insoluble azo dyes on coupling with diazo compounds. Preferred substrates are indoxyl phosphates or substituted indoxyl phosphates. For an assay using β-galactosidase as the enzyme, a suitable substrate is indoxyl-β-D-galactopyranoside. The substrate may be present in a buffered substrate solution at a concentration of about 0.1 to 10 mM, preferably about 1 to 5 mM. Selection of a suitable substrate and the concentration thereof to be used is well within the purview of one skilled in the art.

It has been found that color intensity is magnified if the substrate solution additionally contains a color enhancer. A suitable color enhancer is, for example, a tetrazolium salt. Accordingly, a preferred substrate solution contains about 0.01 to 0.5 mg/mL of p-iodonitrotetrazolium violet or nitroblue tetrazolium in methanol. Most preferably, the indoxyl solution and color enhancing solutions are added sequentially with the enhancer solution preceding the indoxyl solution.

Another aspects of the invention is a test kit or package of materials useful in conducting an assay for the detection of Chlamydia by the method of the invention. The kit may include a filter stack comprising a microporous membrane coated with an anti Chlamydia capture antibody adjacent to an absorbent pad. The membrane may also have a coating of inert protein. The filter stack may be assembled in a housing having access to the membrane for application of assay reagents. The kit additionally includes Proteinase K, a tracer including an enzyme, and a substrate for the enzyme, all preferably supplied as solutions in suitable buffers. The kit may also include other reagents, such as a chaotrope, a specimen transport material and buffers as well as utensils such as vials, droppers, sample collection devices, and the like useful in performing the assay of the invention.

EXAMPLE I

A patient swab sample was placed into 1.5 ml of buffer (sucrose, 68.46 g/L; $K_2HPO_4$, 2.01 g/L; $KH_2PO_4$ 1.01 g/L, Gentamicin, 10 mg/L; Fungizone, 2 mg/L) and vortexed for 3 x 15 seconds. The swab was expressed and 0.6 mL of sample was diluted with 0.15 mL MES buffer (0.5 M, pH 6.0). Proteinase K 0.06 mL of a 10 mg/mL solution in sodium acetate buffer, pH = 4.6) was added and the mixture incubated for 10 minutes at room temperature. The sample was filtered and poured into the sample well of an assay device which includes a BiodyneTMA membrane coated with a polyclonal anti Chlamydia antibody and the sample allowed to absorb onto the coated membrane. A hydrogen peroxide/acetic acid/butanol wash solution (0.3 ml of 15% hydrogen peroxide; 5% glacial acetic acid; 5% butanol; 0.01 M EDTA) was added and allowed to absorb, followed by addition and absorption of 0.15 ml of 0.25 M sodium hydroxide wash solution, pH = 12.5. Detector-antibody conjugate (0.15 mL of 3 ug/0.15 mL monoclonal anti Chlamydia LPS/alkaline phosphatase conjugate in conjugate diluent (200 mM $NaH_2PO_4$, 50 mM Trizma base, 100 mM NaCl, 1% casein, 0.2% sodium azide, 1 mM beta-mercaptoethanol, 1 mM $MgCl_2 \cdot 6H_2O$, 0.1 mM $ZnCl_2$, pH = 7.5) was added and incubated for three minutes at room temperature. Post-conjugate wash solution (0.3 mL of 3.5 M urea, 1% Triton X-705, both in Tris buffered saline (0.05 M Trizma base, 0.9% NaCl, 0.2% sodium azide, pH = 7.2)) was added and allowed to absorb. Substrate A (0.15 mL of 7.8 mM nitrobluetetrazolium in distilled water) was added and allowed to absorb. Substrate B (0.15 mL of $4 \times 10^{-5}$ M Indoxyl phosphate 8 mM levamisole, 0.2% sodium azide in 0.05 M 2-amino-2-methyl-1-propanol, pH = 9.8) was added and incubated for five minutes at room temperature. Stop solution (0.3 mL of 0.15 M citric acid pH = 2.2) was added. A purple spot indicated the presence of Chlamydia antigen in the patient sample.

EXAMPLE II

A total of 1056 clinical samples was assayed in house according to Example I. In 97% of the cases flow through the membrane was unimpeded. Only 5% of the samples gave a false positive result.

EXAMPLE III

The assay of Example I was repeated using the following enzymes in place of Proteinase K.

5

| Enzyme | Comment |
|---|---|
| Rhozyme P-41 | Flow OK, unacceptable readout |
| Rhozyme P-11 | Poor flow, unacceptable readout |
| Bromelain | Fair flow, unacceptable readout |
| Pronase | Fair flow, unacceptable readout |
| Collagenase | Fair flow, decreased signal |
| Subtilisin | Flow OK, unacceptable readout |
| Thermolysin | Poor flow, unacceptable readout |
| Pronase E | Flow OK, false positive signals |
| Nagarase | Flow OK, false positive signals |

EXAMPLE IV

A group of clinical samples taken from 30 volunteers, ten of which were Chlamydia positive and 20 of which were Chlamydia negative, was assayed in-house according to the procedure of Example I. In addition, these samples were also assayed using a modified procedure which deleted proteinase K during the sample processing step. All of the samples treated with proteinase K flowed unimpeded through the assay device. Conversely, 37% of the untreated samples experienced impeded flow through the assay system and produced results which were much more difficult to interpret.

EXAMPLE V

Clinical samples taken from 54 healthy volunteers and known to be Chlamydia negative were divided into two parts and assayed in-house with and without the chaotrope wash. With chaotrope wash, three false positives were observed. In the absence of the chaotrope, 26 false positives were observed.

**Claims**

1. A method for detecting a Chlamydia antigen comprising:
   a) digesting a liquid sample suspected of containing Chlamydia antigen with Proteinase K;
   b) passing the digested sample through a microporous membrane coated with anti Chlamydia antibody to absorb Chlamydia antigen in the sample onto said membrane;
   c) contacting Chlamydia antigen on said membrane with a conjugate of an anti Chlamydia antibody and an enzyme whereby said antibody binds to Chlamydia antigen to give a bound fraction on said membrane;
   d) passing a solution containing a substrate for said enzyme through said membrane whereby said enzyme converts said substrate to a colored product; and
   e) detecting Chlamydia antigen by color on said membrane due to formation of said colored product.

2. The method of Claim 1 further comprising treating the membrane having absorbed antigen thereon with an aqueous solution of an oxidizing agent prior to said contacting step.

3. The method of Claim 1 wherein said membrane is coated with an inert protein.

4. The method of Claim 1 further comprising treating the membrane having said bound fraction thereon with a solution of a chaotropic agent.

5. A method for detecting an antigen in a liquid sample comprising:
   a) digesting a liquid sample suspected of containing an antigen with Proteinase K;

6

b) passing the digested sample through a microporous membrane coated with an antibody whereby said antigen in said sample binds to said antibody on said membrane;

c) contacting antigen absorbed on said membrane with a tracer including a enzyme;

d) passing a solution comprising a substrate for said enzyme through said membrane; and

e) detecting said antigen by a color on said membrane.

6. A method for detecting a Chlamydia antigen comprising:

a) digesting a liquid sample suspected of containing a Chlamydia antigen with a solution containing Proteinase K;

b) filtering the digested sample;

c) passing the filtered sample through a microporous nylon membrane coated with an anti Chlamydia antibody positioned over an absorbent pad to absorb Chlamydia antigen in the sample onto said membrane;

e) contacting Chlamydia antigen on said membrane with a monoclonal anti Chlamydia antibody conjugated to alkaline phosphatase whereby said monoclonal antibody binds to Chlamydia antigen to give a bound fraction on said membrane;

e) washing said membrane having absorbed Chlamydia antigen thereon with a solution comprising urea in a buffer;

f) passing a solution of nitroblue tetrazolium and indoxyl phosphate through said membrane; and

g) detecting Chlamydia antigen in said sample by the appearance of a purple spot on said membrane.

7. A kit of materials for performing an assay for Chlamydia antigen comprising a housing enclosing a microporous membrane coated with an anti Chlamydia antibody, said membrane being adjacent an absorbent pad, Proteinase K, a tracer including an enzyme and a substrate for said enzyme.

8. The kit of Claim 7 further comprising a chaotropic agent.

9. The kit of Claim 7 further comprising an oxidizing agent.

10. The kit of Claim 9 wherein said oxidizing agent is hydrogen peroxide.